# EUROPEAN PATENT APPLICATION

(11) **EP 4 527 830 A1**
(43) Date of publication of application: **26.03.2025**
(21) Application number: 23807937.0
(22) Date of filing: 18.05.2023
(51) Int. Cl.: C07C 51/347, C07C 227/04, C07C 27/10, C07C 209/02, C07C 213/08, C07C 59/01, C07C 229/06, C07C 31/20, C07C 211/02, C07C 215/08

(54) **METHOD FOR PRODUCING FROM FATTY ALCOHOLS MONOMERS FOR PRODUCING VARIOUS SYNTHETIC RESINS**

(30) Priority: 18.05.2022 KR 20220060827; 17.05.2023 KR 20230063745
(71) Applicant: Ahn, Jungoh, Cheongju-si, Chungcheongbuk-do 28116 (KR); Jeon, Woo Young, Cheongju-si, Chungcheongbuk-do 28116 (KR); Jang, Min Jeong, Cheongju-si, Chungcheongbuk-do 28116 (KR); Seo, Sung Hwa, Cheongju-si Chungcheongbuk-do 28116 (KR)
(72) Inventor: YEO, In-Seok, Cheongju-si Chungcheongbuk-do 28116 (KR); AHN, Jungoh, Cheongju-si Chungcheongbuk-do 28116 (KR); JEON, Woo Young, Cheongju-si Chungcheongbuk-do 28116 (KR); JANG, Min Jeong, Cheongju-si Chungcheongbuk-do 28116 (KR); SEO, Sung Hwa, Cheongju-si Chungcheongbuk-do 28116 (KR)
(74) Representative: Schnappauf, Georg
(86) International application number: PCT/KR2023/006804
(87) International publication number: WO 2023/224418

(57) **Abstract**

The present invention relates to a method for producing, from fatty alcohols, various monomers that are used in the production of synthetic resins.

## Description

### Technical Field

The present invention relates to methods of producing various monomers, which are used for the production of synthetic resins, from fatty alcohols.

### Background Art

Bioplatform compounds are produced by biological or chemical transformation based on biomass-derived raw materials and are used in the synthesis of polymeric monomers, new materials, etc.

Among these bioplatform compounds, hydroxyalkanoic acids, aminoalkanoic acids, alkanediols, aminoalkanols, diaminoalkanes, etc. are compounds that are used as monomers for producing synthetic resins such as polyamides and polyesters. However, when alkane or alkanol compounds, which are raw materials for producing these bioplatform compounds, are short-chain compounds having 6 or fewer carbon atoms, they cause cytotoxicity. Therefore, there is a practical difficulty in producing the aforementioned bioplatform compounds using such short-chain alkane compounds through biological methods.

Therefore, there is a need to study or develop technologies capable of biologically producing short-chain bioplatform compounds.

### DISCLOSURE

### Technical Problem

An object of the present invention is to provide methods of producing, from fatty alcohols, monomers for producing synthetic resins.

### Technical Solution

To achieve the above object, one aspect of the present invention provides a method of producing, from fatty alcohol, a monomer for producing synthetic resin, including steps of: synthesizing a dialkyl ether compound from fatty alcohol; fermenting the dialkyl ether compound with a genetically recombinant transformant to produce a dialkyl ether compound functionalized at both ends; and decomposing the dialkyl ether compound functionalized at both ends.

Furthermore, to achieve the above object, another aspect of the present invention provides a method of producing, from fatty alcohol, a monomer for producing synthetic resin, including steps of: synthesizing an asymmetric fatty ether compound from fatty alcohol; fermenting the asymmetric fatty ether compound with a genetically recombinant transformant to produce an asymmetric fatty ether compound functionalized at both ends; and decomposing the asymmetric fatty ether compound functionalized at both ends.

### Advantageous Effects

As described in the present invention, when fatty alcohols are modified into a dialkyl ether compound which is then used as a substrate, it is possible to produce two equivalents of each of various types of monomers carboxylated, hydroxylated, or aminated at both ends through a process of hydrolyzing the functionalized product. It is also possible to functionalize short-chain fatty alcohols, which have been previously difficult to functionalize, and to produce compounds having different functional groups at both ends. Therefore, the present invention has an advantage over the prior art in that it is possible to easily produce various types of monomers for producing synthetic resins.

However, the effects of the present invention are not limited to the effects mentioned above, and other effects not mentioned will be clearly understood by those skilled in the art from the following description.

### Brief Description of Drawings

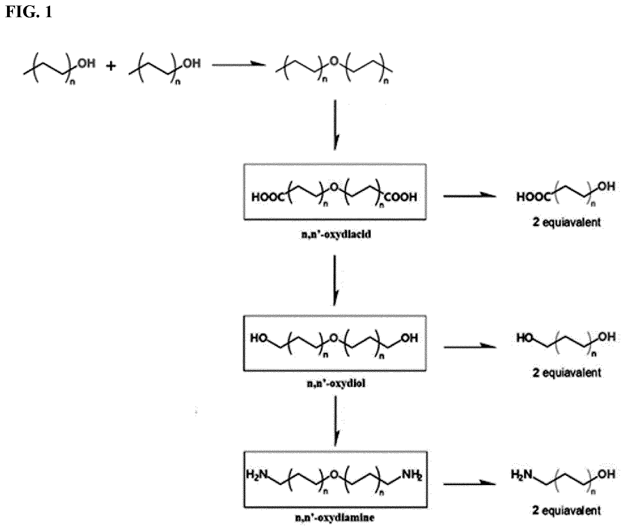

FIG. 1 schematically shows the overall process of producing, from fatty alcohols, monomers for producing synthetic resins according to the present invention.
FIG. 2a depicts graphs showing the results of synthesizing dioctyl ether using biomass-derived 1-octanol, and FIG. 2b depicts a graph showing the results of evaluating the cytotoxicity of the produced dioctyl ether.
FIG. 2c depicts graphs showing the results of synthesizing didodecyl ether using biomass-derived 1-dodecanol, and FIG. 2d depicts a graph showing the results of evaluating the cytotoxicity of the produced didodecyl ether.
FIG. 3 depicts graphs showing the results of evaluating the cytotoxicity of dialkyl ether compounds having different carbon atoms to an recombinant strain for α,ψ-carboxylation of the present invention.
FIGS. 4a and 4b respectively depict graphs showing the results of examining whether dioctyl ether produced from biomass-derived 1-octanol and didodecyl ether synthesized from biomass-derived 1-dodecanol would be functionalized at both ends with carboxyl groups by the recombinant strain for α,ψ-carboxylation of the present invention.
FIG. 5 depicts a graph showing the results of examining whether dioctyl ether functionalized at both ends with carboxyl groups would be functionalized at both ends with hydroxyl groups by a recombinant strain for α,ψ-hydroxylation of the present invention.
FIG. 6 depicts a graph showing the results of examining whether dioctyl ether functionalized at both ends with hydroxyl groups would be functionalized at both ends with amino groups by a recombinant strain for α,ψ-amination of the present invention.
FIG. 7 depicts a graph showing the results of examining whether dioctyl ether functionalized at both ends with carboxyl groups would be hydrolyzed to produce 2 equivalents of 8-hydroxyoctanoic acid.
FIG. 8 depicts a graph showing the results of examining whether dioctyl ether functionalized at both ends with hydroxyl groups would be hydrolyzed to produce 2 equivalents of 1,8-octanediol.
FIG. 9 depicts a graph showing the results of examining whether carboxymethyl 11-carboxyundecyl ether (= 12-(carboxymethoxy)dodecanoic acid), an ethyldodecyl ether compound carboxylated at both ends, would be produced.

### Best Mode

According to one embodiment of the present invention, the present invention relates to a method of producing, from fatty alcohol, a monomer for producing synthetic resin, comprising: step 1 of synthesizing a dialkyl ether compound from fatty alcohol; step 2 of fermenting the dialkyl ether compound with a genetically recombinant transformant to produce a dialkyl ether compound functionalized at both ends; and step 3 of decomposing the dialkyl ether compound functionalized at both ends.

In step 1, the fatty alcohol is a compound in which a saturated hydrocarbon having n carbon atoms is substituted with a hydroxyl group at one end. The number of carbon atoms (n) in the hydrocarbon may be an integer from 3 to 20, for example, an integer from 4 to 18, specifically an integer from 5 to 16, particularly an integer from 6 to 14. In addition, the hydrocarbon may be straight-chain or branched-chain, particularly straight-chain.

In step 2, using a first genetically recombinant transformant, the dialkyl ether compound produced in step 1 may be oxidized at both ends, thereby introducing carboxyl groups at the α,ψ-positions of the dialkyl ether compound. In this case, the first transformant may be a recombinant transformant capable of ψ-oxidation. For example, the first transformant may be a microorganism genetically engineered to block the β-oxidation pathway and overexpress CYP450, CYP450 reductase complex (CPRb), fatty alcohol dehydrogenase (FADH), fatty alcohol oxidase (FAO), fatty aldehyde dehydrogenase (FALDH), or a combination thereof.

In step 2, using a second genetically recombinant transformant or an appropriate catalyst, the dialkyl ether compound functionalized at both ends with carboxyl groups may be further converted into a dialkyl ether compound functionalized at both ends with hydroxyl groups. The second transformant may be a recombinant transformant capable of ψ-reduction. For example, the second transformant may be a microorganism genetically engineered to overexpress carboxylic acid reductase (CAR), 4'-phosphopantetheinyl transferase (Sfp), or a combination thereof.

In step 2, using a third genetically recombinant transformant or an appropriate catalyst, the dialkyl ether compound functionalized at both ends with hydroxyl groups may be further converted into a dialkyl ether compound functionalized at both ends with amino groups. The third transformant may be a recombinant transformant capable of ψ-amination. For example, the third transformant may be a microorganism genetically engineered to overexpress ψ-transaminase (ψ-TA), alanine dehydrogenase (AlaDH), alcohol dehydrogenase (ADH), or a combination thereof.

In step 3, various types of monomers such as hydroxyalkanoic acids, aminoalkanoic acids, alkanediols, aminoalkanols, diaminoalkanes, etc. may be produced through hydrolysis of the ether bond.

Furthermore, in the present invention, various types of monomers produced in step 3 may be used to produce synthetic resins such as polyamide and polyester, and in particular, lactone compounds or lactam compounds may be produced using hydroxyalkanoic acid monomers.

According to another embodiment of the present invention, the present invention relates to a method of producing, from fatty alcohol, a monomer for producing synthetic resin, comprising: step 1' of synthesizing an asymmetric fatty ether compound from fatty alcohol; step 2' of fermenting the asymmetric fatty ether compound with a genetically recombinant transformant to produce an asymmetric fatty ether compound functionalized at both ends; and step 3' of decomposing the asymmetric fatty ether compound functionalized at both ends.

In step 1', the fatty alcohol is a compound in which a saturated or unsaturated aliphatic hydrocarbon having m or n carbon atoms is substituted with a hydroxyl group at one end. The number of carbon atoms (m) in the hydrocarbon may be an integer from 1 to 20, for example, an integer from 1 to 12, specifically an integer from 1 to 6, particularly an integer from 1 to 3. In addition, the number of carbon atoms (n) in the hydrocarbon may be an integer from 3 to 20, for example, an integer from 4 to 18, specifically an integer from 5 to 16, particularly an integer from 6 to 14. Furthermore, the hydrocarbons may be straight-chain or branched-chain, particularly straight-chain.

In step 2', using a first genetically recombinant transformant, the dialkyl ether compound produced in step 1 may be oxidized at both ends, thereby introducing carboxyl groups at the α,ψ-positions of the dialkyl ether compound. In this case, the first transformant may be a recombinant transformant capable of ψ-oxidation, and for example, it may be a microorganism genetically engineered to block the β-oxidation pathway and overexpress CYP450, CYP450 reductase complex (CPRb), fatty alcohol dehydrogenase (FADH), fatty alcohol oxidase (FAO), fatty aldehyde dehydrogenase (FALDH), or a combination thereof.

In step 2', using a second genetically recombinant transformant or an appropriate catalyst, the dialkyl ether compound functionalized at both ends with carboxyl groups may be further converted into a dialkyl ether compound functionalized at both ends with hydroxyl groups. The second transformant may be a genetically recombinant transformant capable of ψ-reduction, and for example, it may be a microorganism genetically engineered to overexpress carboxylic acid reductase (CAR), 4'-phosphopantetheinyl transferase (Sfp), or a combination thereof.

In step 2', using a third genetically recombinant transformant or an appropriate catalyst, the dialkyl ether compound functionalized at both ends with hydroxyl groups may be further converted into a dialkyl ether compound functionalized at both ends with amino groups. The third transformant may be a genetically recombinant transformant capable of ψ-amination, and for example, it may be a microorganism genetically engineered to overexpress ψ-transaminase (ψ-TA), alanine dehydrogenase (AlaDH), alcohol dehydrogenase (ADH), or a combination thereof.

In step 3', various types of monomers such as hydroxyalkanoic acids, aminoalkanoic acids, alkanediols, aminoalkanols, and diaminoalkanes may be produced through hydrolysis of the ether bond.

Furthermore, in the present invention, various types of monomers produced in step 3' may be used to produce synthetic resins such as polyamide and polyester, and in particular, lactone compounds or lactam compounds may be produced using the hydroxyalkanoic acid monomers.

### Mode for Invention

Hereinafter, the present invention will be described in detail.

### 1. First Embodiment - Strategy Mediating Symmetric Dialkyl Ether Compounds

One aspect of the present invention provides a method of producing, from fatty alcohol, a monomer for producing synthetic resin.

The method of the present invention comprises steps of: synthesizing a dialkyl ether compound from fatty alcohol; fermenting the dialkyl ether compound with a genetically recombinant transformant to produce a dialkyl ether compound functionalized at both ends; and decomposing the dialkyl ether compound functionalized at both ends.

### Step 1

First, a dialkyl ether compound is synthesized from fatty alcohol.

The fatty alcohol is a compound in which a saturated hydrocarbon having n carbon atoms is substituted with a hydroxyl group at one end. The number of carbon atoms (n) in the hydrocarbon may be an integer from 3 to 20, for example, an integer from 4 to 18, specifically an integer from 5 to 16, particularly an integer from 6 to 14. In addition, the hydrocarbon may be straight-chain or branched-chain, particularly straight-chain.

The fatty alcohol as described above may be converted into a dialkyl ether compound through the reaction shown in Formula 1 below. Through this reaction, a dialkyl ether compound having 2n carbon atoms may be produced from a fatty alcohol having n carbon atoms. In this case, the produced dialkyl ether compound is a compound in which the same hydrocarbon groups, each having n carbon atoms, are present on both sides of the O atom of the ether bond. Thus, this compound is called a symmetric dialkyl ether compound.

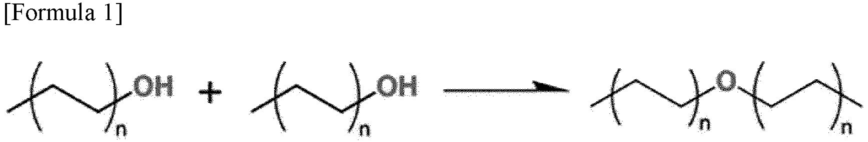

Therefore, dialkyl ether compounds, such as dibutyl ether (C₈H₁₈O), dipentyl ether (C₁₀H₂₂O), dihexyl ether (C₁₂H₂₆O), diheptyl ether (C₁₄H₃₀O), dioctyl ether (C₁₆H₃₄O), dinonyl ether (C₁₈H₃₈O), didecyl ether (C₂₀H₄₂O), diundecyl ether (C₂₂H₄₆O), and didodecyl ether (C₂₄H₅₀O), may be produced from fatty alcohols such as butanol, pentanol, hexanol, heptanol, octanol, nonanol, decanol, undecanol, and dodecanol, respectively.

In addition, the reaction shown in Formula 1 above may be performed through a method well known in the art to which the present invention pertains. For example, the reaction may be performed by carrying out a nucleophilic substitution reaction at a temperature of 130 to 140°C using an acid catalyst such as sulfuric acid (H₂SO₄), without being limited thereto.

### Step 2

The dialkyl ether compound produced in step 1 is fermented with a genetically recombinant transformant to produce a dialkyl ether compound functionalized at both ends.

First, using a first genetically recombinant transformant, as shown in Formula 2 below, the dialkyl ether compound produced in step 1 is oxidized at both ends, thereby introducing carboxyl groups at the α,ψ-positions of the dialkyl ether compound.

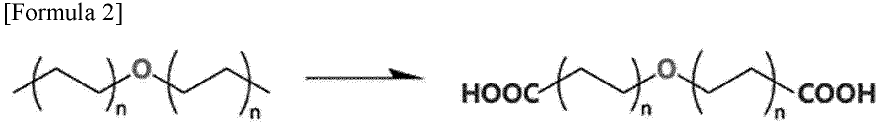

Therefore, the first transformant may be a genetically recombinant transformant capable of ψ-oxidation, and for example, it may be a microorganism genetically engineered to block the β-oxidation pathway and overexpress CYP450, CYP450 reductase complex (CPRb), fatty alcohol dehydrogenase(FADH), fatty alcohol oxidase (FAO), fatty aldehyde dehydrogenase (FALDH), or a combination thereof, without being limited thereto, and any appropriate microorganism genetically engineered by a person skilled in the art to which the present invention pertains to be capable of ψ-oxidation may be used as the first transformant of the present invention without limitation. Here, the microorganism may be a microorganism of the genus *Escherichia, Corynebacterium, Clostridium, Zymonomas, Salmonella, Rhodococcus, Pseudomonas, Bacillus, Lactobacillus, Enterococcus, Alcaligenes, Klesiella, Paenibacillus, Arthrobacter, Brevibacterium, Pichia, Candida, Hansenula, Synechococcus, Synechocystis, Anabaena, Ralstonia, Lactococcus,* or *Saccharomyces,* without being limited thereto.

When the dialkyl ether compound is supplied as a substrate and fermented with the first transformant, the carbon at the ψ-position of the dialkyl ether compound may be oxidized and functionalized with a carboxyl group.

Meanwhile, using a second genetically recombinant transformant or an appropriate catalyst, as shown in Formula 3 below, the dialkyl ether compound functionalized at both ends with carboxyl groups, produced as described above, may be further converted into a dialkyl ether compound functionalized at both ends with hydroxyl groups.

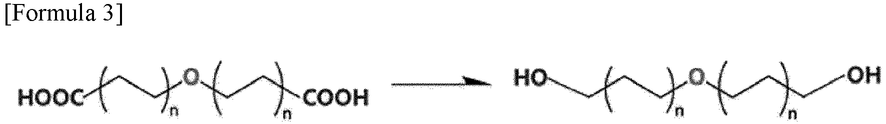

Therefore, the second transformant may be a recombinant transformant capable of ψ-reduction, and for example, it may be a microorganism genetically engineered to overexpress carboxylic acid reductase (CAR), 4'-phosphopantetheinyl transferase (Sfp), or a combination thereof, without being limited thereto, and any appropriate microorganism genetically engineered by a person skilled in the art to which the present invention pertains to be capable of ψ-reduction may be used as the second transformant of the present invention without limitation. Here, the microorganism may be a microorganism of the genus *Escherichia, Corynebacterium, Clostridium, Zymonomas, Salmonella, Rhodococcus, Pseudomonas, Bacillus, Lactobacillus, Enterococcus, Alcaligenes, Klesiella, Paenibacillus, Arthrobacter, Brevibacterium, Pichia, Candida, Hansenula, Synechococcus, Synechocystis, Anabaena, Ralstonia, Lactococcus,* or *Saccharomyces,* without being limited thereto.

When the dialkyl ether compound functionalized at both ends with carboxyl groups is supplied as a substrate and fermented with the second transformant, the carboxyl group at the ψ-position of the dialkyl ether compound functionalized at both ends with carboxyl groups may be reduced and functionalized into a hydroxyl group.

Furthermore, using a third genetically recombinant transformant or an appropriate catalyst, as shown in Formula 4 below, the dialkyl ether compound functionalized at both ends with hydroxyl groups, produced as described above, may be further converted into a dialkyl ether compound functionalized at both ends with amino groups.

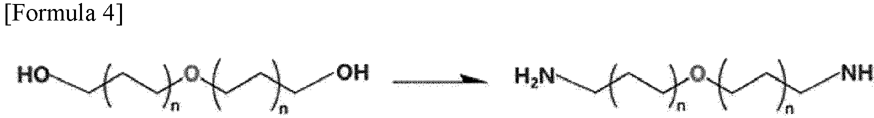

Therefore, the third transformant may be a recombinant transformant capable of ψ-amination, and for example, it may be a microorganism genetically engineered to overexpress ψ-transaminase (ψ-TA), alanine dehydrogenase (AlaDH), alcohol dehydrogenase (ADH), or a combination thereof, without being limited thereto, and any appropriate microorganism genetically engineered by a person skilled in the art to which the present invention pertains to be capable of ψ-amination may be used as the third transformant of the present invention without limitation. Here, the microorganism may be a microorganism of the genus *Escherichia, Corynebacterium, Clostridium, Zymonomas, Salmonella, Rhodococcus, Pseudomonas, Bacillus, Lactobacillus, Enterococcus, Alcaligenes, Klesiella, Paenibacillus, Arthrobacter, Brevibacterium, Pichia, Candida, Hansenula, Synechococcus, Synechocystis, Anabaena, Ralstonia, Lactococcus,* or *Saccharomyces,* without being limited thereto.

When the dialkyl ether compound functionalized at both ends with hydroxyl groups is supplied as a substrate and fermented with the third transformant, the hydroxyl group at the ψ-position of the dialkyl ether compound functionalized at both ends with hydroxyl groups may be aminated and functionalized with an amino group.

### Step 3

The dialkyl ether compound functionalized at both ends, produced in step 2, is decomposed. The decomposition is the hydrolysis of the ether bond of the dialkyl ether compound functionalized at both ends. Such hydrolysis of the ether bond may be performed through a method widely known in the art to which the present invention pertains, and for example, it may be performed by reaction at a temperature of 200°C in the presence of an acid catalyst such as sulfuric acid (H₂SO₄), without being limited thereto.

Such hydrolysis of the ether bond may be performed on all types of dialkyl ether compounds functionalized at both ends that may be produced in step 2. Therefore, a dialkyl ether compound having 2n carbon atoms and functionalized at both ends with carboxyl groups may be hydrolyzed to produce 2 equivalents of a hydroxyalkanoic acid with n carbon atoms or 2 equivalents of an aminoalkanoic acid with n carbon atoms, or a dialkyl ether compound having 2n carbon atoms and functionalized at both ends with hydroxyl groups may be hydrolyzed to produce 2 equivalents of an alkanediol with n carbon atoms or 2 equivalents of an aminoalkanol with n carbon atoms, or a dialkyl ether compound having 2n carbon atoms and functionalized at both ends with amino groups may be hydrolyzed to produce 2 equivalents of an aminoalkanol having n carbon atoms or 2 equivalents of a diaminoalkane having n carbon numbers.

Various types of monomers such as hydroxyalkanoic acids, aminoalkanoic acids, alkanediol, aminoalkanol, diaminoalkane, etc., produced as described above, may be useful for the production of synthetic resins such as polyamide, polyester, etc. In particular, hydroxyalkanoic acid may be converted into a lactone compound through the reaction described in previous documents (for example, Pyo et al., Green Chem., 22: 4450-4455

(2020), etc.), and such a lactone compound may also be converted into a lactam through the reaction described in several previous documents (Rankic et al., J. Org. Chem., 82(23): 12791-12797 (2017), Decker et al., Tetrahedron, 60(21): 4567-4678 (2004), etc.), and thus these compounds may be utilized more diversely.

### 2. Second Embodiment - Strategy Mediating Asymmetric Fatty Ether Compounds

Another aspect of the present invention provides a method of producing, from fatty alcohol, a monomer for producing synthetic resin.

The method of the present invention comprises steps of: synthesizing an asymmetric fatty ether compound from fatty alcohol; fermenting the asymmetric fatty ether compound with a genetically recombinant transformant to produce an asymmetric fatty ether compound functionalized at both ends; and decomposing the asymmetric fatty ether compound functionalized at both ends.

### Step 1'

First, an asymmetric fatty ether compound is synthesized from fatty alcohol.

The fatty alcohol is a compound in which a saturated or unsaturated aliphatic hydrocarbon having m or n carbon atoms is substituted with a hydroxyl group at one end. The number of carbon atoms (m) in the hydrocarbon may be an integer from 1 to 20, for example, an integer from 1 to 12, specifically an integer from 1 to 6, particularly an integer from 1 to 3. In addition, the number of carbon atoms (n) in the hydrocarbon may be an integer from 3 to 20, for example, an integer from 4 to 18, specifically an integer from 5 to 16, particularly an integer from 6 to 14. Furthermore, the hydrocarbons may be straight-chain or branched-chain, particularly straight-chain.

The fatty alcohol described above may be converted into an asymmetric fatty ether compound through the reaction shown in Formula 5 below. Through this reaction, a fatty ether compound having m+n carbon atoms may be produced from a fatty alcohol having m carbon atoms and a fatty alcohol having n carbon atoms. Here, m and n are different numbers. The resulting fatty ether compound is a compound in which the hydrocarbon group having m carbon atoms and the hydrocarbon group having n carbon atoms are present on both sides centered on the O atom of the ether bond. Thus, this compound is called an asymmetric fatty ether compound.

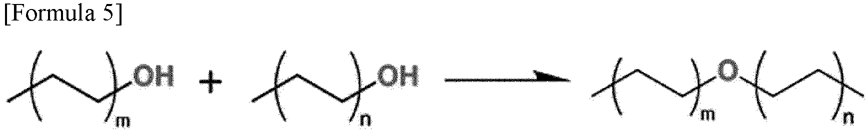

For example, when methanol, an alcohol with m=1, is reacted with fatty alcohols such as butanol, pentanol, hexanol, heptanol, octanol, nonanol, decanol, undecanol, and dodecanol, asymmetric dialkyl ether compounds such as methyl butyl ether (or 1-methoxybutane (C₅H₁₂O)), methyl pentyl ether (1-methoxypentane (C₆H₁₄O)), methyl hexyl ether (1-methoxyhexane (C₇H₁₆O)), methyl heptyl ether (1-methoxyheptane (C₈H₁₈O)), methyl octyl ether (1-methoxyoctane (C₉H₂₀O)), methyl nonyl ether (1-methoxynonane (C₁₀H₂₂O)), methyl decyl ether (1-methoxydecane (C₁₁H₂₄O)), methyl undecyl ether (1-methoxyundecane (C₁₂H₂₆O)), and methyl dodecyl ether (1-methoxydodecane (C₁₃H₂₈O)) may be produced, respectively.

Furthermore, when ethanol, an alcohol with m=2, is reacted with fatty alcohols such as butanol, pentanol, hexanol, heptanol, octanol, nonanol, decanol, undecanol, and dodecanol, asymmetric dialkyl ether compounds such as ethyl butyl ether (1-ethoxybutane (C₆H₁₄O)), ethyl pentyl ether (1-ethoxypentane (C₇H₁₆O)), ethyl hexyl ether (1-ethoxyhexane (C₈H₁₈O)), ethyl heptyl ether (1-ethoxyheptane (C₉H₂₀O)), ethyl octyl ether (1-ethoxyheptane (C₁₀H₂₂O)), ethyl nonyl ether (1-ethoxynonane (C₁₁H₂₄O)), ethyl decyl ether (1-ethoxydecane (C₁₂H₂₆O)), ethyl undecyl ether (1-ethoxyundecane (C₁₃H₂₈O)), and ethyl dodecyl ether (1-ethoxydodecane (C₁₄H₃₀O)) may be produced, respectively.

Moreover, alcohols with m≥3 may also be reacted with fatty alcohols such as butanol, pentanol, hexanol, heptanol, octanol, nonanol, decanol, undecanol, and dodecanol to produce various asymmetric dialkyl ether compounds.

In addition, the reaction shown in Formula 5 above may be performed through a method widely known in the art to which the present invention pertains, and for example, it may be performed by carrying out a nucleophilic substitution reaction at a temperature of 130 to 140°C in the presence of an acid catalyst such as sulfuric acid (H₂SO₄). Alternatively, the asymmetric dialkyl ether compounds may also be produced by reacting a fatty alcohol with dimethyl sulfate ((CH₃)₂SO₄) as shown in Formula 6 below or with diethyl sulfate ((CH₃CH₂)₂SO₄) as shown in Formula 7 below, without being limited thereto.

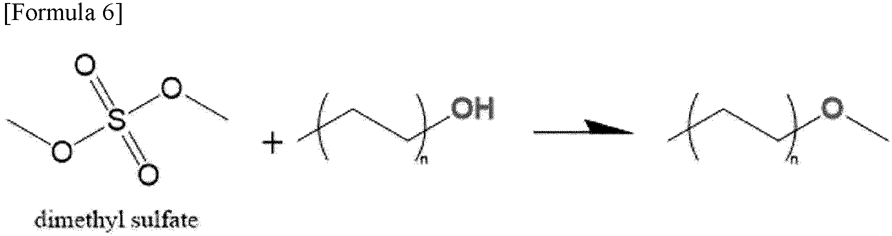

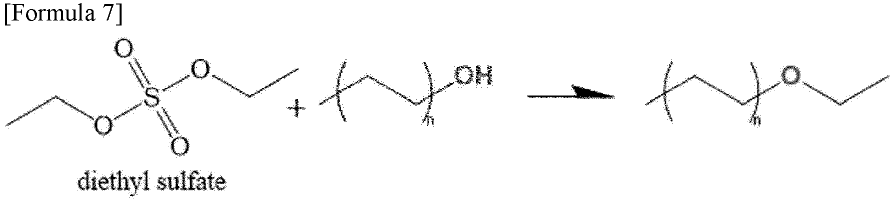

Meanwhile, as shown in Table 1 below, if the number of carbon atoms (n) in the fatty alcohol used for the synthesis of the symmetric dialkyl ether is 14 or more, a problem arises in that the melting point of the resulting dialkyl ether compound with 2n carbon atoms is higher than 45°C, making it difficult to achieve functionalization at both ends through the biological conversion reaction used in the present invention. As a result, the strategy of using symmetric dialkyl ether compounds has a problem in that it is difficult to produce hydroxyalkanoic acids, aminoalkanoic acids, alkanediols, aminoalkanols, diaminoalkanes, etc. which have 14 or more carbon atoms. However, as described in Table 1 below, in the case of asymmetric fatty ether compounds, even ethyl hexadecyl ether having a total carbon number of 18, produced using ethanol with m=2 and hexadecanol with n=16, has a melting point of only about 18°C, and thus the biological conversion reaction used in the present invention easily occurs. Therefore, the strategy of using asymmetric fatty ether compounds has the advantage of being able to produce hydroxyalkanoic acids, aminoalkanoic acids, alkanediols, aminoalkanols, diaminoalkanes, etc. which have more carbon atoms than those in the strategy of using symmetric dialkyl ether compounds.

**[Table 1]**

| Symmetric dialkyl ether | Melting point (°C) | Asymmetric fatty ether | Melting point (°C) |
|---|---|---|---|
| Dioctyl ether (CH₃(CH₂)₇-O-(CH₂)₇CH₃) | -7.6 | Ethyl octyl ether (1-ethoxyoctane) (CH₃(CH₂)-O-(CH₂)₇CH₃) | 12.5 |
| Didecyl ether (CH₃(CH₂)₉-O-(CH₂)₉CH₃) | 16 | Ethyl decyl ether (1-ethoxydecane) (CH₃(CH₂)-O-(CH₂)₉CH₃) | 12.5<, <16 |
| Diundecyl ether (CH₃(CH₂)₁₀-O-(CH₂)₁₀CH₃) | 24 | Ethyl undecyl ether (1-ethoxyundecane) (CH₃(CH₂)-O-(CH₂)₁₀CH₃) | |
| Didodecyl ether (CH₃(CH₂)₁₁-O-(CH₂)₁₁CH₃) | 32 | Ethyl dodecyl ether (1-ethoxydodecane) (CH₃(CH₂)-O-(CH₂)₁₁CH₃) | |
| Ditetradecyl ether (CH₃(CH₂)₁₃-O-(CH₂)₁₃CH₃) | 45 | Ethyl tetradecyl ether (1-ethoxytetradecane) (CH₃(CH₂)-O-(CH₂)₁₃CH₃) | 16 |
| Dihexadecyl ether (CH₃(CH₂)₁₅-O-(CH₂)₁₅CH₃) | 55 | Ethyl hexadecyl ether (1-Ethoxyhexadecane) (CH₃(CH₂)-O-(CH₂)₁₅CH₃) | 18 |

### Step 2'

The asymmetric fatty ether compound produced in step 1' is fermented with a genetically recombinant transformant to produce an asymmetric fatty ether compound functionalized at both ends.

This step may be performed in the same manner as in step 2 of the first embodiment. First, using a first genetically recombinant transformant, the asymmetric fatty ether compound may be oxidized at both ends, thereby introducing carboxyl groups at the α,ψ-positions of the asymmetric fatty ether compound.

In addition, using a second genetically recombinant transformant or an appropriate catalyst, the asymmetric fatty ether compound functionalized at both ends with carboxyl groups, produced as described above, may be further functionalized at both ends with hydroxyl groups.

Furthermore, using a third genetically recombinant transformant or an appropriate catalyst, the asymmetric fatty ether compound functionalized at both ends with hydroxyl groups, produced as described above, may be further functionalized at both ends with amino groups.

Step 2' of functionalizing both ends of the asymmetric fatty ether compound involves the same biological or chemical method as in step 2 of the first embodiment, and thus detailed explanation thereof is as described above in the "step 2" section of the first embodiment and will be omitted.

### Step 3'

The asymmetric fatty ether compound functionalized at both ends, produced in step 2' is decomposed. The decomposition is also hydrolysis of the ether bond of the asymmetric fatty ether compound functionalized at both ends, and such hydrolysis of the ether bond may be performed through a method widely known in the art to which the present invention pertains, and may be performed in the same manner as in step 3 of the first embodiment.

Therefore, such hydrolysis of the ether bond may be performed on all types of asymmetric fatty ether compounds functionalized at both ends that may be produced in step 2'. Therefore, an asymmetric aliphatic ether compound having m+n carbon atoms and functionalized at both ends with carboxyl groups may be hydrolyzed to produce either 1 equivalent of a hydroxyalkanoic acid having m carbon atoms and 1 equivalent of a hydroxyalkanoic acid having n carbon atoms, or 1 equivalent of an aminoalkanoic acid having m carbon atoms and 1 equivalent of an aminoalkanoic acid having n carbon atoms. Alternatively, an asymmetric fatty ether compound having m+n carbon atoms and functionalized at both ends with hydroxyl groups may be hydrolyzed to produce either 1 equivalent of an alkanediol having m carbon atoms and 1 equivalent of an alkanediol having n carbon atoms, or 1 equivalent of an aminoalkanol having m carbon atoms and 1 equivalent of an aminoalkanol having n carbon atoms. Alternatively, an asymmetric fatty ether compound having m+n carbon atoms and functionalized at both ends with amine groups may be hydrolyzed to produce either 1 equivalent of an aminoalkanol having m carbon atoms and 1 equivalent of an aminoalkanol having n carbon atoms, or 1 equivalent of a diaminoalkane having m carbon atoms and 1 equivalent of a diaminoalkane having n carbon atoms.

Various types of monomers such as hydroxyalkanoic acids, aminoalkanoic acids, alkanediols, aminoalkanols, diaminoalkanes, etc. produced as described above may be useful for the production of synthetic resins such as polyamides and polyesters. In particular, hydroxyalkanoic acid may be converted into a lactone compound through the reaction described in previous documents (e.g., Pyo et al., Green Chem., 22: 4450-4455

(2020), etc.), and such lactone compounds may also be converted into lactams through a reaction known in several previous documents (Rankic et al., J. Org. Chem., 82(23): 12791-12797 (2017), Decker et al., Tetrahedron, 60(21): 4567-4678 (2004), etc.), and thus these compounds may be utilized more diversely.

Hereinafter, the present invention will be described in detail by way of examples.

However, the following examples are intended to illustrate the present invention in detail, and the content of the present invention is not limited by the following examples.

### [Example 1]

### Synthesis of dialkyl ether compounds from fatty alcohols

### [1-1] Synthesis of dioctyl ether

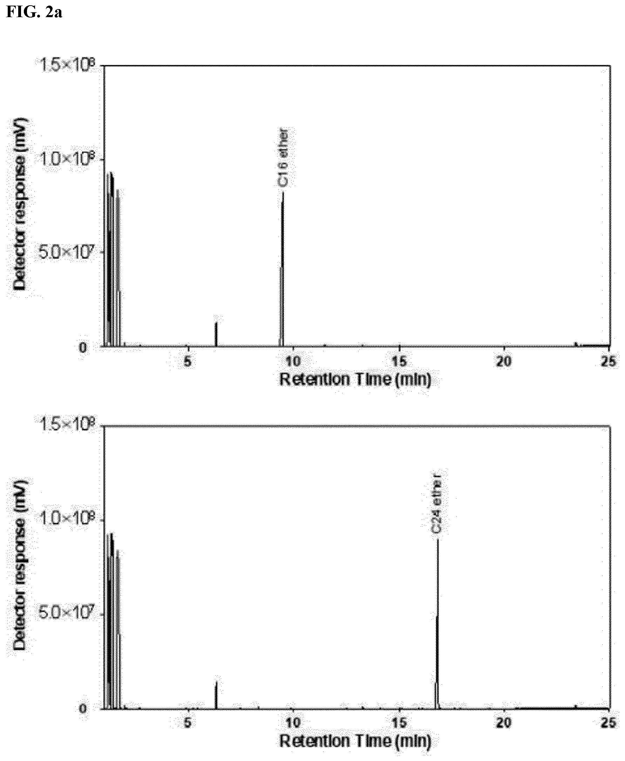

Sulfuric acid was added to biomass-derived 1-octanol to be 0.01 mol%, and then synthesis was carried out by reaction at 200°C for 20 hours. Dioctyl ether was separated from the reaction mixture by fractional distillation under a reduced pressure of 20 Torr. The purity of the separated dioctyl ether was analyzed by a gas chromatography-mass spectrometry (GC-MS) system equipped with a quadrupole electron selective ionization detector (EI) operating at 70 eV. An Agilent HP-5MS column (length: 30 m, inner diameter: 0.25 mm, and film thickness: 0.25 µm) was used with a split ratio of 10:1. Helium (flow rate: 1.2 mL/min) was used as the carrier gas, and the oven temperature was raised from 100°C to 320°C (10°C/min). The synthesized substrate and an equal volume of diethyl ether were used to extract the substrate, and tetradecane was used as an internal standard. The purity of the synthesized substrate was 97% or more as shown in FIG. 2a, and the impurity was unreacted 1-octanol that did not participate in the reaction.

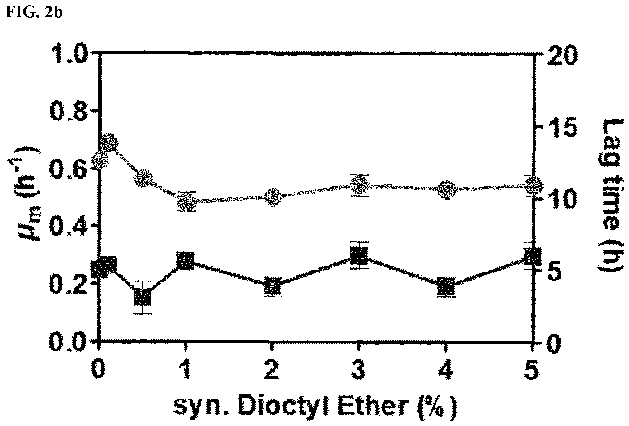

The cytotoxicity of the dioctyl ether synthesized as described above was analyzed. 200 µl of a mixed solution of YPD medium (10 g/L yeast extract, 20 g/L Bacto peptone, 20 g/L glucose) and each concentration (0, 0.25, 0.5, 1, 2, 3, 4, or 5%) of dioctyl ether was added to each well of a 48-well plate. After 2 µl of a culture of a recombinant *Candida tropicalis* Ct6 strain cultured overnight in YPD medium at 30°C and 200 rpm was further added to each well, the absorbance at 600 nm was measured using a plate reader at 30°C for 24 hours or more at 15-minute intervals. The maximum specific growth rate (µₘ) and lag time were calculated using the Gompertz growth equation. As a result, as shown in FIG. 2b, it was confirmed that the synthesized dioctyl ether had almost no cytotoxicity.

### [1-2] Synthesis of didodecyl ether

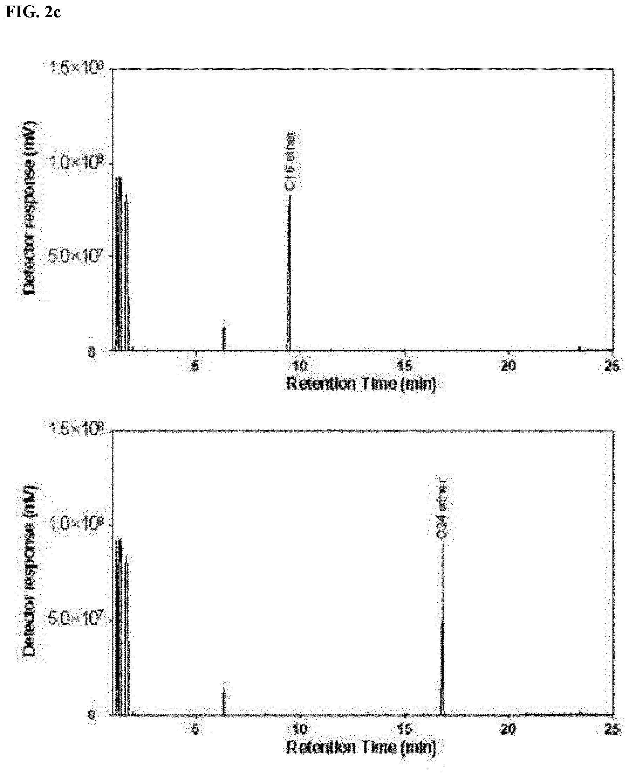

Sulfuric acid was added to biomass-derived 1-dodecanol to be 0.01 mol%, and then synthesis was carried out by reaction at 200°C for 20 hours. Didodecyl ether was separated from the reaction mixture by fractional distillation under a reduced pressure of 20 Torr. The purity of the separated didodecyl ether was analyzed by a gas chromatography-mass spectrometry (GC-MS) system equipped with a quadrupole electron selective ionization detector (EI) operating at 70 eV. An Agilent HP-5MS column (length: 30 m, inner diameter: 0.25 mm, and film thickness: 0.25 µm) was used with a split ratio of 10:1. Helium (flow rate: 1.2 mL/min) was used as the carrier gas, and the oven temperature was raised from 100°C to 320°C (10°C/min). The synthesized substrate and an equal volume of diethyl ether were used to extract the substrate, and tetradecane was used as an internal standard. The purity of the synthesized substrate was 97% or more as shown in FIG. 2c, and the impurity was unreacted 1-dodecanol that did not participate in the reaction.

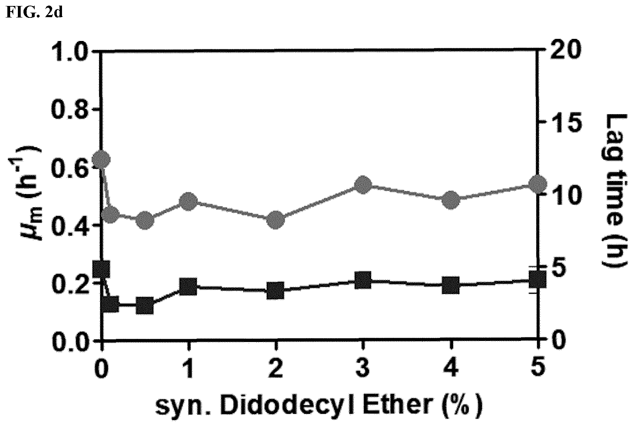

The cytotoxicity of the didodecyl ether synthesized as described above was analyzed. 200 µl of a mixed solution of YPD medium and each concentration (0, 0.25, 0.5, 1, 2, 3, 4, or 5%) of didodecyl ether was added to each well of a 48-well plate. After 2 µl of a culture of a recombinant *Candida tropicalis* Ct6 strain cultured overnight in YPD medium at 30°C and 200 rpm was further added to each well, the absorbance at 600 nm was measured using a plate reader at 33°C for 24 hours or more at 15-minute intervals. The maximum specific growth rate and lag time were calculated using the Gompertz growth equation. As a result, as shown in FIG. 2d, it was confirmed that the synthesized didodecyl ether had almost no cytotoxicity.

### [Example 2]

### Production of dialkyl ether compounds functionalized at both ends with carboxyl groups from alkyl ether compounds

### [2-1] Preparation of recombinant strain for α,ψ-carboxylation

A recombinant *Candida tropicalis* Ct6 strain having the genotype shown in Table 2 below, described in Jeon et al., Green Chem, 21, 6491-6501 (2019), was used.

**[Table 2]**

| **Recombinant strain** | **Genotype** |
|---|---|
| *C. tropicalis* Ct6 | *ura3*/*ura3 pox2Δ::CYP52A19*/*POX2 pox4*/*pox4 pox5Δ::CPRB-URA3*/*pox5* |

The *Candida tropicalis* Ct6 strain was kept in 15% glycerol at -70°C, and the stock strain was plated on YPD agar medium (10 g/L yeast extract, 20 g/L Bacto peptone, 20 g/L glucose, and 20 g/L agarose) and cultured overnight at 30°C.

### [2-2] Toxicity test for recombinant strain for α,ψ-carboxylation

Prior to the biotransformation of alkyl ether compounds, a toxicity test for the recombination strain for α,ψ-carboxylation according to the concentration of the substrate was performed.

First, YPD medium (10 g/L yeast extract, 20 g/L Bacto peptone, 20 g/L glucose) was mixed with different concentrations (0, 0.25, 0.5, 1, 2, 3, 4, and 5%) of ether compounds with different carbon numbers (dibutyl ether, dipentyl ether, dihexyl ether, diheptyl ether, dioctyl ether, didecyl ether, diundecyl ether, didodecyl ether; each obtained from TCI). Then, 200 µL of the mixed solution of YPD medium and each reagent was seeded into each well of a 48-well plate. The next day, 2 µL of the recombinant strain for α,ψ-carboxylation of Example [2-1], which has been inoculated into 2 mL of YPD medium the day before and cultured at 30°C and 200 rpm for one day, was seeded into each well containing the mixed solution, and the growth of the recombinant strain for α,ψ-carboxylation was measured using a plate reader (Tecan Corp.) for 24 hours at 15-minute intervals. Using the measured results, values approximating the Gompertz growth equation were calculated and the maximum specific growth rate (µₘ) and lag time (µ) were calculated.

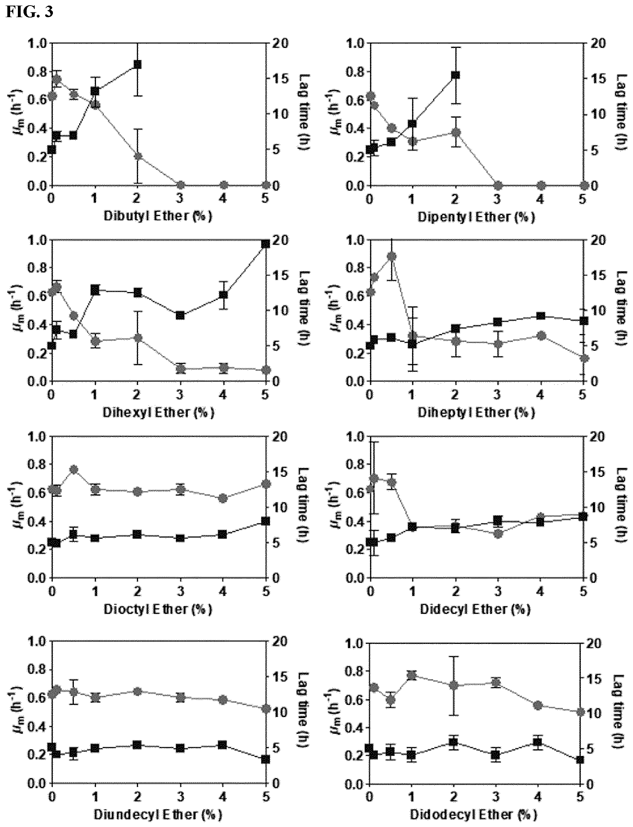

As a result, as shown in FIG. 3, it was confirmed that ether compounds with 14 or more carbon atoms inhibited the growth of the strain less than ether compounds with 12 or fewer carbon atoms, and in particular, ether compounds with 16 or more carbon atoms hardly inhibited cell growth even at a high substrate concentration of 5%.

### [2-3] Confirmation of α,ψ-carboxylation of dialkyl ether compounds

First, to confirm whether dialkyl ether compounds can be biologically α,ψ-carboxylated, a laboratory-scale 5-L culture experiment was conducted. The recombinant strain for α,ψ-carboxylation prepared in Example [2-1] was inoculated into 2 mL of YPD medium and pre-cultured at 30°C and 200 rpm for 24 hours. The pre-culture was added to a 2-L baffled flask containing 200 mL of YPD medium and cultured at 30°C and 200 rpm for 24 hours. The next day, all of the 200-mL culture was added to a fermentor containing 1.8 L of a medium having the composition shown in Table 3 below to make the final volume up to 2 L, and the final composition of the 2-L culture is shown in Table 3 below.

**[Table 3]**

| **Component** | **Concentration (g/L)** |
|---|---|
| Glycerol | 80 |
| CaCl₂·2H₂O | 0.1 |
| NaCl | 0.1 |
| Yeast extract | 20 |
| (NH₄)₂SO₄ | 8 |
| KH₂PO₄ | 2 |
| Trace elements | 1 mL |
| Antifoam | 0.3 mL |
| MgSO₄·7H₂O | 1 |

When the glycerol contained in the initial culture was completely consumed, a feeding medium (600 g/L glucose, 30 g/L NH₄Cl) was continuously added at a rate of 0.167 mL/min. To activate the ψ-oxidation pathway of the recombinant strain for α,ψ-carboxylation, 3 mL of dodecane (TCI Corp.) was added along with the feeding medium. Three hours after addition of the dodecane, 10 mL of each of substrates (dibutyl ether, dipentyl ether, dihexyl ether, diheptyl ether, dioctyl ether, didecyl ether, diundecyl ether, and didodecyl ether) was added to the culture which was then incubated. For the initial cell growth, the pH was maintained at 6, and when the substrate was added, the pH was adjusted to 7.5. The pH of the culture was adjusted using a 6N NaOH solution. Dissolved oxygen was adjusted by controlling the agitation speed and maintained at 30% or higher, and the temperature of the fermentor was maintained at 30°C (33°C for diundecyl ether and didodecyl ether). The culture at 4 and 6 hours after addition of the substrate was subjected to acid treatment, and the substrate and product for GC-MS analysis were extracted with an equal volume of diethyl ether. Then, the substrate and the product were analyzed using a gas chromatography-mass spectrometry (GC-MS) system equipped with a quadrupole electron selective ionization detector operating at 70 eV, and at this time, an Agilent HP-5MS column (length: 30 m, internal diameter: 0.25 mm, and film thickness: 0.25 µm) was used with a split ratio of 10:1. Helium was used as the carrier gas (flow rate: 1.2 mL/min), and the oven temperature was raised from 100°C to 320°C (10°C/min). Tetradecane was used as an internal standard, and carboxylated dialkyl ether compounds were identified by the GC-MS fragmentation profile as there were no commercially available reagents.

As a result, it was confirmed that all the dialkyl ether compounds used as the substrates were converted into the respective α,ψ-carboxylated products, and the conversion rates are shown in Table 4 below.

**[Table 4]**

| **Substrate** | **Conversion rate(%)** | |
|---|---|---|
| | **4 hours after substrate addition** | **6 hours after substrate addition** |
| Dibutyl ether (C8) | 65.91 ± 0.17 | 67.66 ± 0.52 |
| Dipentyl ether (C10) | 44.80 ± 0.31 | 70.79 ± 0.23 |
| Dihexyl ether (C12) | 54.54 ± 1.06 | 73.14 ± 0.55 |
| Diheptyl ether (C14) | 80.66 ± 4.89 | 100 |
| Dioctyl ether (C16) | 90.17 ± 0.62 | 100 |
| Didecyl ether (C20) | 65.37 ± 1.35 | 76.24 ± 1.42 |
| Diundecyl ether (C22) | 97.31 ± 1.44 | 100 |
| Didodecyl ether (C24) | 75.58 ± 3.00 | 100 |

**[2-4] Confirmation of α,ψ-carboxylation of biomass-derived dialkyl ether compounds** Next, to confirm whether biomass-derived dialkyl ether compounds can also be biologically α,ψ-carboxylated by the recombinant strain for α,ψ-carboxylation prepared in Example [2-1], α,ψ-carboxylation of dialkyl ether compounds was performed in the same manner as in Example [2-3], using dioctyl ether and didodecyl ether synthesized in Example 1 as substrates.

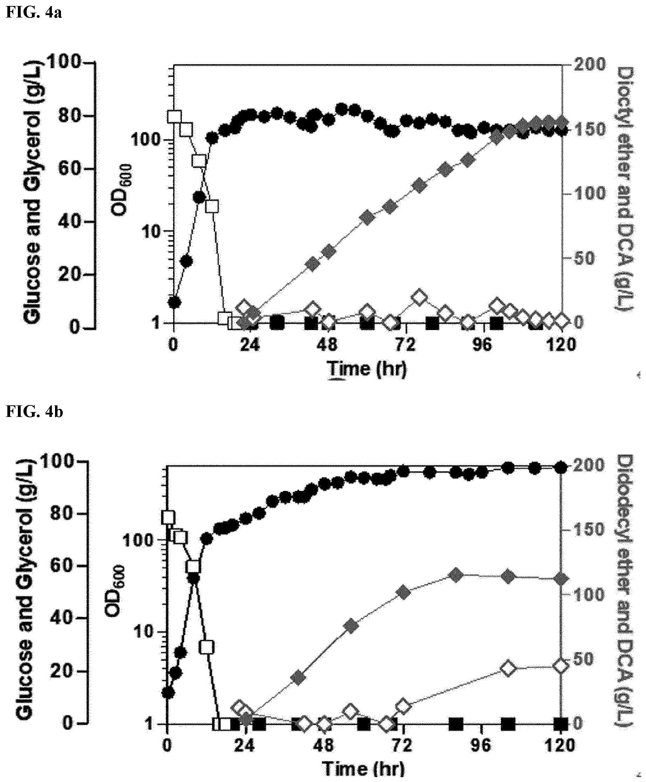

As a result, as shown in FIG. 4a, it was confirmed that 160 g/L of α,ψ-carboxylated dioctyl ether was produced after 120 hours, and as shown in FIG. 4b, it was confirmed that 110 g/L of α,ψ-carboxylated didodecyl ether was produced after 85 hours.

### [Example 3]

### Production of dialkyl ether compounds functionalized at both ends with hydroxyl groups from dialkyl ether compounds functionalized at both ends with carboxyl groups

### [3-1] Preparation of recombinant strain for α,ψ-hydroxylation

To place the carboxylic acid reductase (CAR) gene (WP_00582584.1) from *Mycobacteroides abscessus* and the 4'-phosphopantetheinyl transferase (Sfp) gene (WP_00234549.1) from *Bacillus* sp. in an operon structure under the Tac promoter, the nucleotide sequence of SEQ ID NO: 1 was synthesized by ATUM (USA), and the synthesized gene was inserted into the BamHI and HindIII sites of pJ281 (ATUM). The resulting recombinant vector pJ281-CAR-Sfp was transfected into the *Escherichia coli* MG1665(DE3) strain (Novagen), thereby constructing a recombinant strain for α,ψ-hydroxylation.

The recombinant *E. coli* strain for α,ψ-hydroxylation was maintained in 15% glycerol at -70°C, and the stock strain was plated on LB agar medium (5 g/L yeast extract, 10 g/L Bacto tryptone, 10 g/L NaCl, 20 g/L agarose, and 50 mg/L kanamycin) containing kanamycin and cultured overnight at 37°C.

### [3-2] Confirmation of α,ψ-hydroxylation of dialkyl ether compounds functionalized at both ends with carboxyl groups

Next, to confirm the biological α,ψ-hydroxylation of dialkyl ether compounds functionalized at both ends with carboxyl groups, the culture in which dioctyl ether synthesized in Example 1 was biotransformed in Example [2-4] was centrifuged to remove cells, and whether α,ψ-hydroxylated dioctyl ether would be produced was examined using the supernatant as a substrate solution.

For this purpose, the recombinant strain for α,ψ-hydroxylation prepared in Example [3-1] was inoculated into 2-mL LB medium (5 g/L yeast extract, 10 g/L Bacto tryptone, 10 g/L NaCl, and 50 mg/L kanamycin) containing kanamycin and pre-cultured for one day at 37°C and 200 rpm. The pre-culture was added to a 2-L baffled flask containing 200 mL LB medium containing kanamycin and cultured for one day at 37°C and 200 rpm. The next day, all of the 200 mL culture was added to a fermentor containing 1.8 L of a medium having the components shown in Table 5 below to make the final volume up to 2 L. The final composition of the 2-L culture is shown in Table 5 below.

**[Table 5]**

| **Component** | **Concentration (g/L)** |
|---|---|
| Glucose | 15 |
| Galactose | 15 |
| Glycerol | 80 |
| Soy peptone | 10 |
| Yeast extract | 10 |
| (NH₄)₂ SO₄ | 5 |
| KH₂PO₄ | 6 |
| Trace element | 1 mL |
| Antifoam | 0.3 mL |
| MgSO₄·7H₂O | 2 |
| Kanamycin | 0.05 |

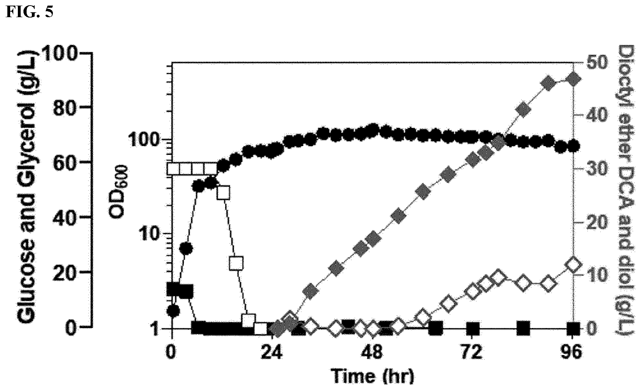

When the carbon sources, including glucose and glycerol, contained in the initial culture, were completely consumed, a feeding medium (800 g/L glucose, 30 g/L NH₄Cl) and a substrate (α,ψ-carboxylated dioctyl ether produced in Example [2-4]) were continuously added at a rate of 0.167 mL/min. For initial cell growth, the pH was maintained at 7 using ammonia water, and when the substrate was added, the pH was maintained at 7.5 using 6N NaOH. Dissolved oxygen was adjusted by controlling the agitation speed and maintained at 30% or more. The temperature of the fermentor was maintained at 35°C, and the components of the culture were analyzed in the same manner as in Example [2-3]. As a result, as shown in FIG. 5, it was confirmed that the α,ψ-carboxylated dioctyl ether were hydroxylated at both ends, indicating that α,ψ-hydroxylated dioctyl ether was produced.

### [Example 4]

### Production of dialkyl ether compounds functionalized at both ends with amino groups from dialkyl ether compounds functionalized at both ends with hydroxyl groups

### [4-1] Preparation of recombinant strain for α,ψ-amination

The alcohol dehydrogenase (ADH) gene (WP_130157107.1) from *Aeribacillus pallidus,* the ψ-transaminase (ψ-TA) gene (WP_011135573.1) from *Chromobacterium violaceum,* and the alanine dehydrogenase (AlaDH) gene (WP_003243280.1) from *Bacillus subtilis* were synthesized in the form of the nucleotide sequence of SEQ ID NO: 2 as an operon structure by ATUM Corp. (USA), and the synthesized gene was inserted into the SmaI and HindIII sites of the pJ281 vector (ATUM). The resulting recombinant vector was transfected into the *Escherichia coli* MG1665(DE3) strain (Novagen), thereby constructing a recombinant strain for α,ψ-amination.

The recombinant *E. coli* strain for α,ψ-amination was maintained in 15% glycerol at -70°C. The stock strain was plated on LB agar medium (5 g/L yeast extract, 10 g/L Bacto tryptone, 10 g/L NaCl, 20g/L agarose, and 50 mg/L kanamycin) containing kanamycin and cultured overnight at 37°C.

### [4-2] Confirmation of α,ψ-amination of dialkyl ether compounds functionalized at both ends with hydroxyl groups

Next, to confirm the biological α,ψ-amination of dialkyl ether compounds functionalized at both ends with hydroxyl groups, the culture in which α,ψ-hydroxylated dioctyl ether was produced by biotransformation in Example [3-2] was centrifuged to remove the supernatant, and methanol and water were sequentially added to the precipitate to induce crystallization of α,ψ-hydroxylated dioctyl ether. Then, the crystals filtered through cellulose paper were redissolved in methanol, and 0.5% activated carbon was added thereto to induce decolorization. Water was then added to induce recrystallization, and the crystals were filtered again through cellulose paper, washed with distilled water, and dried at 80°C to thereby isolate and purify α,ψ-hydroxylated dioctyl ether with a purity of 99% or higher. Whether α,ψ-aminated dioctyl ether would be produced was examined using the α,ψ-hydroxylated dioctyl ether as a substrate.

For this purpose, the recombinant strain for α,ψ-amination prepared in Example [4-1] was batch-cultured in a 5-L fermentor with a medium having the components shown in Table 5 below. Then, the culture was centrifuged, the supernatant was removed, and the cells were stored at -70°C. Using the stored strain, a biotransformation mixture was prepared by dissolving the composition shown in Table 6 below in a 0.1 M phosphate buffer (pH 8.0), and 50 mL of the biotransformation mixture was cultured in a 500-mL baffled flask at 37°C for 24 hours or more, while the components of the culture were analyzed in the same manner as in Example [2-3].

**[Table 6]**

| **Component** | **Concentration (g/L)** |
|---|---|
| Recombinant strain for α,ψ-amination (DCW) | 70 |
| NH₄Cl | 7.5 |
| DMSO | 20% (*v*/*v*) |
| L-Alanine | 6.7 |
| Benzylamine | 6.7 |
| α,ψ-hydroxylated dioctyl ether | 10 |

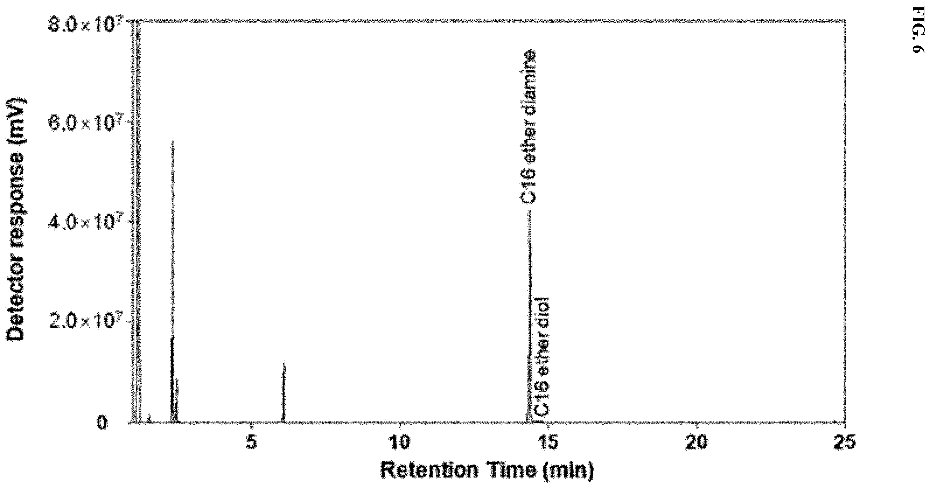

As a result, as shown in FIG. 6, it was confirmed that the α,ψ-hydroxylated dioctyl ether were aminated at both ends, indicating that α,ψ-aminated dioctyl ether was produced.

### [Example 5]

### Hydrolysis of dialkyl ether compounds functionalized at both ends

The α,ψ-carboxylated dioctyl ether produced in Example [2-4] or the α,ψ-hydroxylated dioctyl ether produced in Example [3-2] was dissolved in 1N sulfuric acid to a concentration of 10 g/L, and reacted in a high-pressure reactor at 200°C for 1 hour or more, and the components of the reaction mixture were analyzed in the same manner as in Example [2-3].

In the case of dioctyl ether, the culture in which the dioctyl ether synthesized in Example 1 was biotransformed was centrifuged to remove the cells, and the pH of the supernatant was lowered to 4 by adding H₂SO₄ to induce precipitation of α,ψ-carboxylated dioctyl ether. Then, the precipitated α,ψ-carboxylated dioctyl ether was collected by filtration through cellulose paper. The α,ψ-carboxylated dioctyl ether collected as described above was completely dissolved in acetic acid at 95°C, and then the temperature was lowered back to room temperature to induce crystallization of the α,ψ-carboxylated dioctyl ether. Then, the crystals were filtered again through cellulose paper and washed with water to thereby isolate and purify the α,ψ-carboxylated dioctyl ether with a purity of 86.79%. This was used as a substrate for hydrolysis.

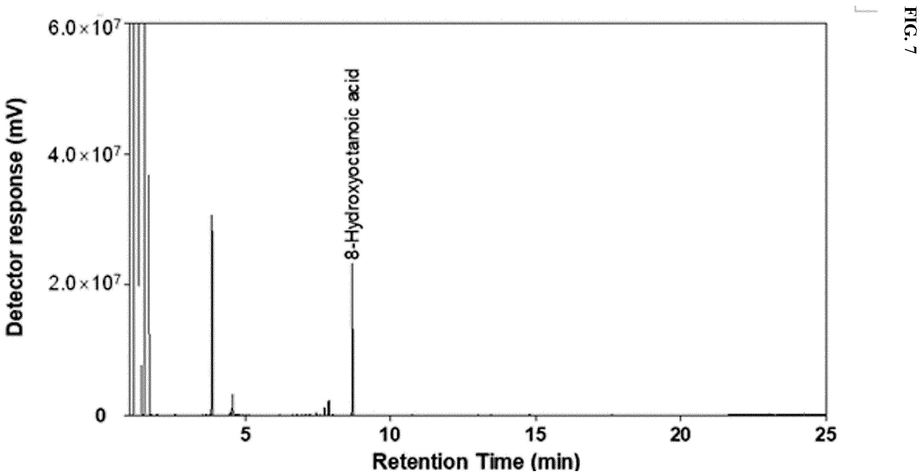

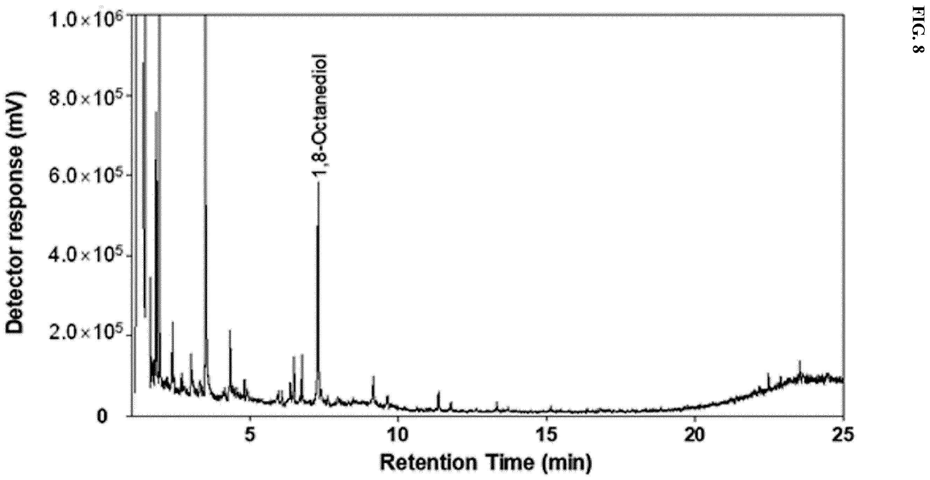

As a result, as shown in FIG. 7, it was confirmed that 8-hydroxyoctanoic acid was produced from α,ψ-carboxylated dioctyl ether, and as shown in FIG. 8, it was confirmed that 1,8-octanediol was produced from α,ψ-hydroxylated dioctyl ether.

For the hydrolysis of α,ψ-aminated dioctyl ether, 1N sulfuric acid was added to the biotransformation mixture containing α,ψ-aminated dioctyl ether prepared in Example 4, and the mixture was allowed to react in a high-pressure reactor at 200°C for 1 hour or more. Then, the components of the reaction mixture were analyzed in the same manner as in Example [2-3]. As a result, it was confirmed that 8-aminooctanol was produced.

### [Example 6]

### Production of monomer, for producing synthetic resin, from asymmetric dialkyl ether compound

### [6-1] Synthesis of ethyldodecyl ether (1-ethoxydodecane)

Toluene and biomass-derived 1-dodecanol were mixed with NaOH at room temperature, reacted at 80°C for 1 hour, and then cooled. Diethyl sulfate ((CH₃CH₂)₂SO₄) was added thereto at 30°C and mixed for 1 hour. Then, the temperature was raised to 60°C and the mixture was allowed to react for 6 hours. The reaction mixture was filtered to remove salts and then distilled to obtain ethyldodecyl ether (1-ethoxydodecane).

The purity of the product was analyzed using a gas chromatography-mass spectrometry (GC-MS) system equipped with a quadrupole electron selective ionization detector operating at 70 eV, and at this time, an Agilent HP-5MS column (length: 30 m, inner diameter: 0.25 mm, and film thickness: 0.25 µm) was used with a split ratio of 10:1. Helium (flow rate: 1.2 mL/min) was used as the carrier gas, and the oven temperature was raised from 100°C to 320°C (10°C/min). The synthesized substrate and an equal volume of diethyl ether were used to extract the substrate, and tetradecane was used as an internal standard. The purity of the synthesized ethyldodecyl ether was 97% or more, and the impurity was unreacted 1-decanol that did not participate in the reaction.

### [6-2] Production of asymmetric dialkyl ether compound functionalized at both ends with carboxyl groups from ethyldodecyl ether

Fermentation was performed using the recombinant strain for α, ψ-carboxylation prepared in Example [2-1] while supplying ethyldodecyl ether produced in Example [5-1] as a substrate, in the same manner as in Example [2-3].

After adding the substrate ethyldodecyl ether, the culture was acid-treated and extraction of the substrate and product for GC-MS analysis was performed using an equal volume of diethyl ether. Then, the substrate and product were analyzed using a gas chromatography-mass spectrometry (GC-MS) system equipped with a quadrupole electron selective ionization detector operating at 70 eV, and at this time, an Agilent HP-5MS column (length: 30 m, inner diameter: 0.25 mm, and film thickness: 0.25 um) was used with a split ratio of 10:1. Helium (flow rate: 1.2 mL/min) was used as the carrier gas, and the oven temperature was raised from 100°C to 320°C (10°C/min). Tetradecane was used as an internal standard, and the carboxylated dialkyl ether compound was identified by the GC-MS fragmentation profile because there were no commercially available reagents.

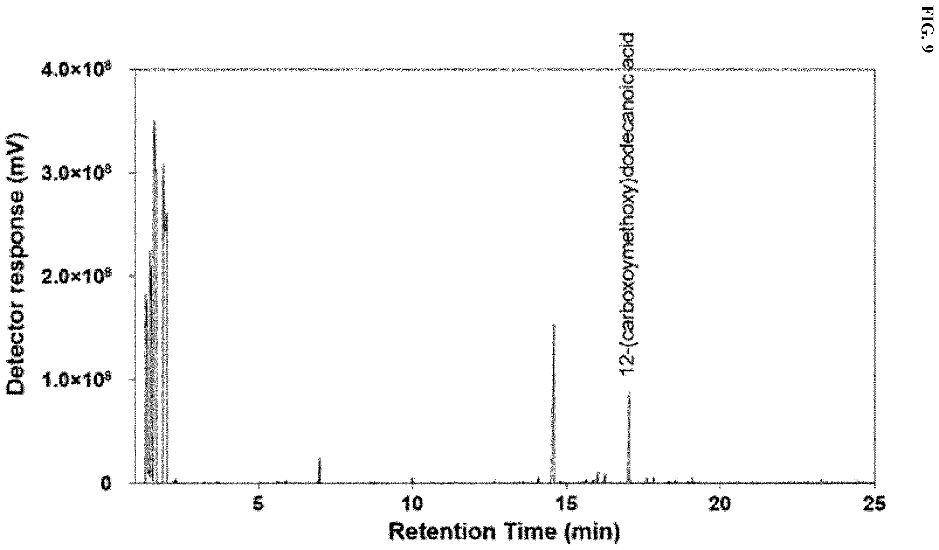

As a result, as shown in FIG. 9, it was confirmed that carboxymethyl 11-carboxyundecyl ether (= 12-(carboxymethoxy)dodecanoic acid), an ethyldodecyl ether compound carboxylated at both ends, was produced.

### [6-3] Utilization of asymmetric dialkyl ether compounds functionalized at both ends with carboxyl groups

Ethyldodecyl ether functionalized at both ends with carboxyl groups (= carboxymethyl 11-carboxyundecyl ether) produced in Example [5-2] can be further functionalized at both ends with hydroxyl or amino groups in the same manner as in Example 3 and Example 4. Furthermore, these asymmetric dialkyl ether compounds functionalized at both ends with carboxyl, hydroxyl, or amino groups can be hydrolyzed in the same manner as in Example 5 to produce various monomers for plastics, such as 12-hydroxydodecanoic acid, 12-aminododecanol, and 1,12-dodecanediol.

Meanwhile, the 12-hydroxydodecanoic acid produced as described above can be converted to a lactone through the reaction described in Pyo et al., Green Chem., 22: 4450-4455 (2020). For example, 12-hydroxydodecanoic acid is converted into the corresponding acid chloride by reaction with thionyl chloride in a suitable solvent such as dichloromethane or chloroform, and the resulting acid chloride solution is treated with a suitable base such as triethylamine or pyridine to neutralize the hydrogen chloride produced during the reaction. Then, a solution of a suitable lactone ring-closure agent such as triethyl orthoformate or diisopropyl azodicarboxylate is added to the reaction mixture which is then is stirred at room temperature for a considerable time. Then, the mixture is heated under reflux at a temperature of about 80°C to 100°C for a considerable time to promote the lactonization of the acid chloride into the lactone form. After completion of the reaction, the reaction mixture is cooled and the unreacted acid chloride is quenched by adding water or a suitable aqueous solution. Then, dodecanolactone is extracted from the reaction mixture using a suitable solvent such as ethyl acetate or dichloromethane, and purified by distillation or recrystallization to produce high-purity dodecanolactone.

Furthermore, the dodecanolactone produced as described above can be converted into dodecanelactam through the reaction described in several previous documents (Rankic et al., J. Org. Chem., 82(23): 12791-12797 (2017), Decker et al., Tetrahedron, 60(21): 4567-4678 (2004), etc.). For example, dodecanolactone is dissolved in a suitable solvent such as methanol or ethanol, an ammonium hydroxide solution is added to the dodecanolactone solution, and the mixture is stirred at room temperature for a considerable time. Then, the reaction mixture is heated under reflux at a temperature of about 120°C to 140°C for a considerable time to promote the cyclization of the lactone into the lactam form. After completion of the reaction, the mixture is cooled and the pH is adjusted to about 7 using hydrochloric acid. Then, dodecanelactam is extracted from the reaction mixture using a suitable solvent such as ethyl acetate or dichloromethane, and purified by recrystallization or chromatography to produce high-purity dodecanelactam.

Although preferred embodiments of the present invention have been described by way of example, the scope of the present invention is not limited to these specific embodiments, and those skilled in the art will appreciate that appropriate modifications are possible without departing from the scope of the claims of the present invention.

### Industrial Applicability

The present invention relates to methods of producing various monomers, which are used for the production of synthetic resins, from fatty alcohols.

## Claims

1. A method for producing a monomer for producing synthetic resin, comprising steps of:
fermenting a dialkyl ether compound with a genetically recombinant transformant to produce a dialkyl ether compound functionalized at both ends; and
decomposing the dialkyl ether compound functionalized at both ends.

2. A method for producing a monomer for producing synthetic resin, comprising steps of:
fermenting an asymmetric fatty ether compound with a genetically recombinant transformant to produce an asymmetric fatty ether compound functionalized at both ends; and
decomposing the asymmetric fatty ether compound functionalized at both ends.

3. The method of claim 1 or 2, wherein the dialkyl ether compound or the asymmetric fatty ether compound is synthesized from fatty alcohol.

4. The method of claim 3, wherein the fatty alcohol is a straight-chain fatty alcohol having 6 to 20 carbon atoms.

5. The method of claim 1, wherein the dialkyl ether compound is at least one selected from the group consisting of dihexyl ether (C₁₂H₂₆O), diheptyl ether (C₁₄H₃₀O), dioctyl ether (C₁₆H₃₄O), dinonyl ether (C₁₈H₃₈O), didecyl ether (C₂₀H₄₂O), diundecyl ether (C₂₂H₄₆O), and didodecyl ether (C₂₄H₅₀O), and
the asymmetric fatty ether compound is at least one selected from the group consisting of methyl butyl ether (or 1-methoxy butane (C_{S}H₁₂O)), methyl pentyl ether (1-methoxy pentane (C₆H₁₄O)), methyl hexyl ether (1-methoxy hexane (C₇H₁₆O)), methyl heptyl ether (1-methoxy heptane (C₈H₁₈O)), methyl octyl ether (1-methoxy octane (C₉H₂₀O)), methyl nonyl ether (1-methoxy nonane (C₁₀H₂₂O)), methyl decyl ether (1-methoxy decane (C₁₁H₂₄O)), methyl undecyl ether (1-methoxy undecane (C₁₂H₂₆O)), methyl dodecyl ether (1-methoxy dodecane (C₁₃H₂₈O)), ethyl butyl ether (1-ethoxy butane (C₆H₁₄O)), ethyl pentyl ether (1-ethoxy pentane (C₇H₁₆O)), ethyl hexyl ether (1-ethoxy hexane (C₈H₁₈O)), ethyl heptyl ether (1-ethoxy heptane (C₉H₂₀O)), ethyl octyl ether (1-ethoxy heptane (C₁₀H₂₂O)), ethyl nonyl ether (1-ethoxy nonane (C₁₁H₂₄O)), ethyl decyl ether (1-ethoxy decane (C₁₂H₂₆O)), ethyl undecyl ether (1-ethoxy undecane (C₁₃H₂₈O)), and ethyl dodecyl ether (1-ethoxy dodecane (C₁₄H₃₀O)).

6. The method of claim 1 or 2, wherein the dialkyl ether compound or the asymmetric fatty ether compound functionalized at both ends is a dialkyl ether compound or asymmetric fatty ether compound substituted at both ends with any one of a carboxyl group, a hydroxyl group, and an amino group.

7. The method of claim 1 or 2, wherein the genetically recombinant transformant comprises a microorganism in which a β-oxidation pathway is blocked and CYP450, CYP450 reductase complex (CPRb), and fatty alcohol oxidase (FAO) are overexpressed.

8. The method of claim 1 or 2, wherein the decomposition is hydrolysis.

9. The method of claim 1 or 2, wherein the monomer for producing synthetic resin is an aliphatic alkane substituted at one end with any one of a carboxyl group, a hydroxyl group, and an amino group, and substituted at the other end with any one of a hydroxyl group and an amino group.
